# EUROPEAN PATENT APPLICATION

(11) **EP 3 845 635 A1**
(43) Date of publication of application: **07.07.2021**
(21) Application number: 19856084.9
(22) Date of filing: 29.08.2019
(51) Int. Cl.: C12N 5/077, A61K 35/33, A61P 9/00, A61P 9/04, A61P 9/10

(54) **METHOD FOR PRODUCING FIBROBLAST, AND G-CSF-POSITIVE FIBROBLAST MASS**

(30) Priority: 29.08.2018 JP 2018160898
(71) Applicant: Metcela Inc., Tsuruoka-shi, Yamagata, 997-0002 (JP)
(72) Inventor: IWAMIYA, Takahiro, Tsuruoka-shi, Yamagata 997-0015 (JP); OSUGI, Tomoyuki, Tsuruoka-shi, Yamagata 997-0015 (JP); SUZUKI, Masaya, Tsuruoka-shi, Yamagata 997-0015 (JP)
(74) Representative: Jones, Nicholas Andrew
(86) International application number: PCT/JP2019/033835
(87) International publication number: WO 2020/045547

(57) **Abstract**

Provided is a method of producing CD106-positive and/or CD90-positive fibroblasts. Also provided is a cell population of G-CSF-positive fibroblasts. The method of producing CD106-positive and/or CD90-positive fibroblasts include the step of culturing fibroblasts in the presence of at least one selected from the group consisting of TNF-α and IL-4 to increase the number of CD106-positive and/or CD90-positive fibroblasts.

## Description

### TECHNICAL FIELD

The present invention mainly relates to methods of producing CD106-positive and/or CD90-positive fibroblasts. The present invention further relates to pharmaceutical compositions for treatment of heart diseases comprising the fibroblasts. The present invention also relates to cell populations comprising G-CSF-positive fibroblasts.

### BACKGROUND ART

Fibroblasts are one of the interstitial cells present in various living organs. Fibroblasts play roles in secretion of growth factors and cytokines, and production and decomposition of extracellular matrices in response to inflammation or injury in organs or tissues, as well as in control of functions of organs and tissues, and maintenance of homeostasis of microenvironment via various cell-to-cell interactions. In another aspect, fibroblasts also maintain pathological microenvironment in diseases such as fibrosis and cancers and allow them to progress. In general, fibroblasts are known to be spindle cells having many cytoplasmic projections and to significantly differentially express genes and proteins depending on the organs to which they are localized (see Non-patent Document 1).

As for fibroblasts, the present inventors have previously found that fibroblasts that are positive for Vascular cell adhesion molecule-1 (VCAM-1, CD106) can be used to provide functional cardiac cell sheets (see Patent Document 1).

### PRIOR ART DOCUMENT

### PATENT DOCUMENTS

Patent Document 1: WO2016/006262
Patent Document 2: WO2018/155651

### NON-PATENT DOCUMENT

Non-patent Document 1: Chang, H. Y. et al., Diversity, topographic differentiation, and positional memory in human fibroblasts. Proc. Natl. Acad. Sci. 99, 12877-12882 (2002)

### SUMMARY OF THE INVENTION

### PROBLEMS TO BE SOLVED BY THE INVENTION

In Patent Document 1, for example, cell sorting techniques using CD106 as a marker are used to obtain a sufficient number of CD106-positive fibroblasts. However, some fibroblasts may produce very small amounts (cell numbers) of CD106-positive fibroblasts.

On the other hand, the present inventors have developed injectables for treatment of heart diseases and filed a patent application therefor (see Patent Document 2). In Patent Document 2, the present inventors have found that preferred fibroblasts that are effective as injectables are positive for CD106, as well as positive for CD90.

An object of the present invention is to provide a novel method of producing a fibroblast that is positive for CD106 and/or CD90.

### MEANS FOR SOLVING THE PROBLEMS

In order to achieve the above object, the present inventors have studied to find that culturing of fibroblasts in the presence of at least one selected from the group consisting of Tumor Necrosis Factor-alpha (TNF-α) known as a factor that induces hemorrhagic necrosis of tumor and Interleukin-4 (IL-4) known for its role in onset of allergic reactions leads to increase in the number of CD106-positive and/or CD90-positive fibroblasts, thereby completing the present invention.

The present invention includes the following first aspect (invention A):
(A1) A method of producing CD106-positive and/or CD90-positive fibroblasts, comprising the step of:
   culturing fibroblasts in the presence of at least one selected from the group consisting of TNF-α and IL-4 to increase the number of CD106-positive and/or CD90-positive fibroblasts;
(A2) The method according to (A1), wherein the fibroblasts to be cultured are obtained from an adult;
(A3) The method according to (A1) or (A2), wherein the CD106-positive and/or CD90-positive fibroblasts are positive for CD 106 and CD90;
(A4) The method according to any one of (A1) to (A3), further comprising the step of enriching the CD106-positive and/or CD90-positive fibroblasts; and
(A5) The method according to any one of (A4), wherein the enrichment is made using an anti-CD106 antibody and/or an anti-CD90 antibody.

The present invention also includes the following second aspect (invention B):
(B1) A cell population comprising fibroblasts comprising CD106-positive adult fibroblasts;
(B2) The cell population according to (B1), wherein the percentage (number of cells) of the CD106-positive adult fibroblasts in the fibroblasts is 3.36% or more, preferably 5% or more; and wherein more preferably 7% or more of the fibroblasts are positive for CD90, CD106, and G-CSF;
(B3) The cell population according to (B1) or (B2), wherein the adult fibroblasts are positive for CD106 and CD90;
(B4) The cell population according to any one of (B1) to (B3), wherein the adult fibroblasts are stimulated with TNF-α and/or IL-4;
(B5) A pharmaceutical composition comprising the cell population according to any one of (B1) to (B4) and a pharmaceutically acceptable excipient;
(B6) The pharmaceutical composition according to (B5), wherein the composition is a therapeutic agent for ischemic heart disease; and
(B7) The pharmaceutical composition according to (B5) or (B6), wherein the composition is administered to an organ from which the adult fibroblasts are derived.

The present invention includes the following third aspect (invention C):
(C1) A method of producing G-CSF-positive fibroblasts, comprising the step of:
   culturing fibroblasts in the presence of at least one selected from the group consisting of TNF-α and IL-4 to increase the expression level of G-CSF and thereby increase the number of G-CSF-positive fibroblasts;
(C2) The method according to (C1), wherein the fibroblasts to be cultured is obtained from an adult;
(C3) The method according to (C1) or (C2), further comprising, after the culturing, the step of enriching the G-CSF fibroblasts;
(C4) The method according to any one of (C1) to (C3), wherein the enrichment comprises a sorting step using an anti-CD 106 antibody and/or an anti-CD90 antibody;
(C5) A cell population comprising isolated CD106-positive and G-CSF-positive fibroblasts; and
(C6) The cell population according to (C5), wherein the percentage (number of cells) of G-CSF-positive fibroblasts in fibroblasts is 6.75% or more.

The present invention further includes the following aspect (invention D):
(D1) An injectable composition for treatment of heart diseases, comprising G-CSF-positive fibroblasts;
(D2) The injectable composition according to (D1), wherein the percentage (number of cells) of the G-CSF-positive fibroblasts in fibroblasts is 6.75% or more;
(D3) A method of treating heart diseases, comprising the steps of:
   preparing a fibroblast population comprising G-CSF-positive fibroblasts; and
   injecting the fibroblast population into a necrosed cardiac tissue region or a peripheral region thereof and/or into a coronary artery; and
(D4) The method according to (D3), wherein the percentage (number of cells) of the G-CSF-positive fibroblasts in fibroblasts is 6.75% or more.

### EFFECTS OF THE INVENTION

The present invention provides a novel method of producing CD106-positive and/or CD90-positive fibroblasts. The present invention further provides a cell population comprising G-CSF-positive fibroblasts.

### BRIEF DESCRIPTION OF THE DRAWINGS

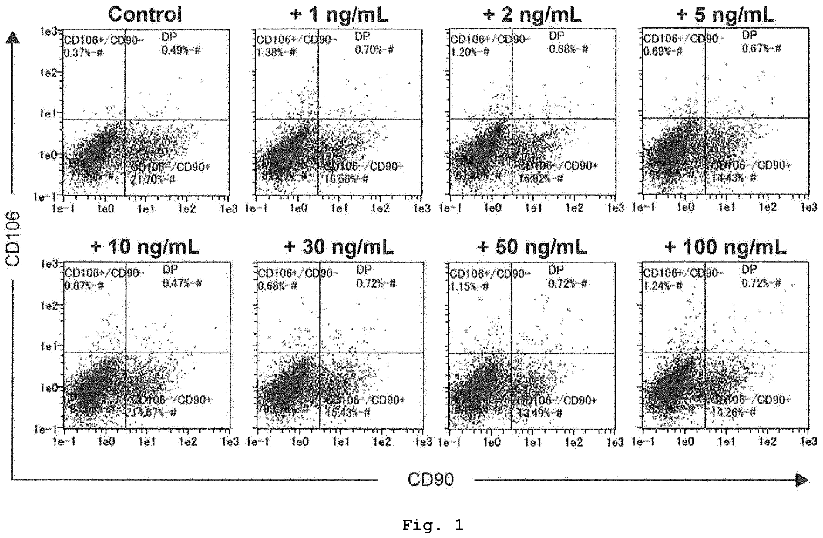

FIG. 1 shows the flow cytometry analysis of adult cardiac fibroblasts with addition of TNF-α.

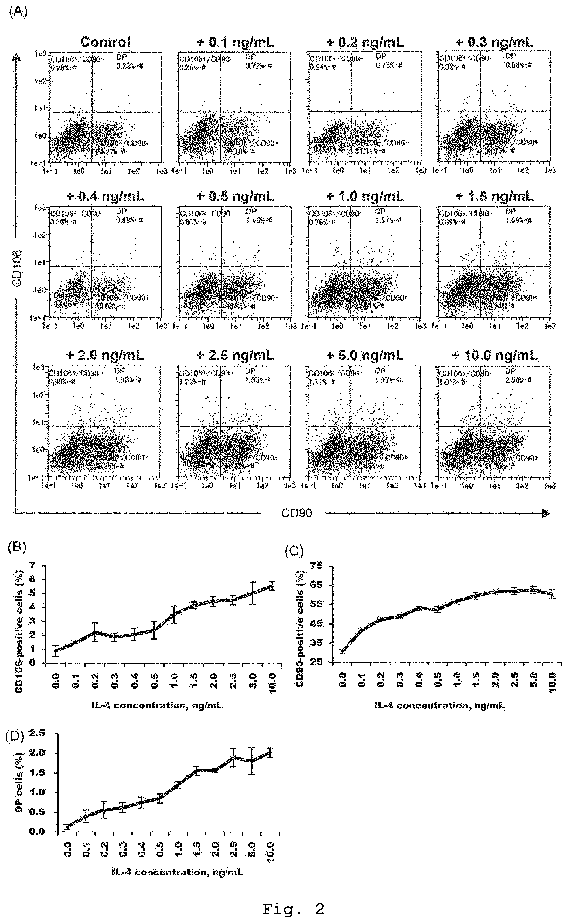

FIG. 2 shows: (A) shows the flow cytometry analysis of adult cardiac fibroblasts with addition of IL-4. (B) shows a graph representing the percentage (%) of CD106-positive cells in adult cardiac fibroblasts in the presence of various concentrations of IL-4 (N = 3). (C) shows a graph representing the percentage (%) of CD90-positive cells in adult cardiac fibroblasts in the presence of various concentrations of IL-4 (N = 3). (D) shows a graph representing the percentage (%) of CD106-positive and CD90-positive cells in adult cardiac fibroblasts in the presence of various concentrations of IL-4 (N = 3).

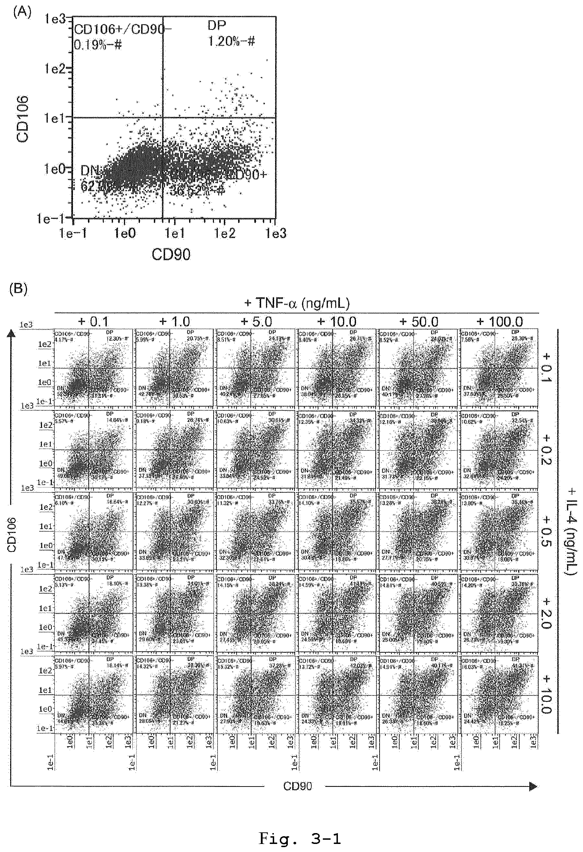

FIG. 3-1 shows: (B) shows the flow cytometry analysis of adult cardiac fibroblasts with addition of two agents, TNF-α and IL-4. (A) shows a control without addition of either of them.

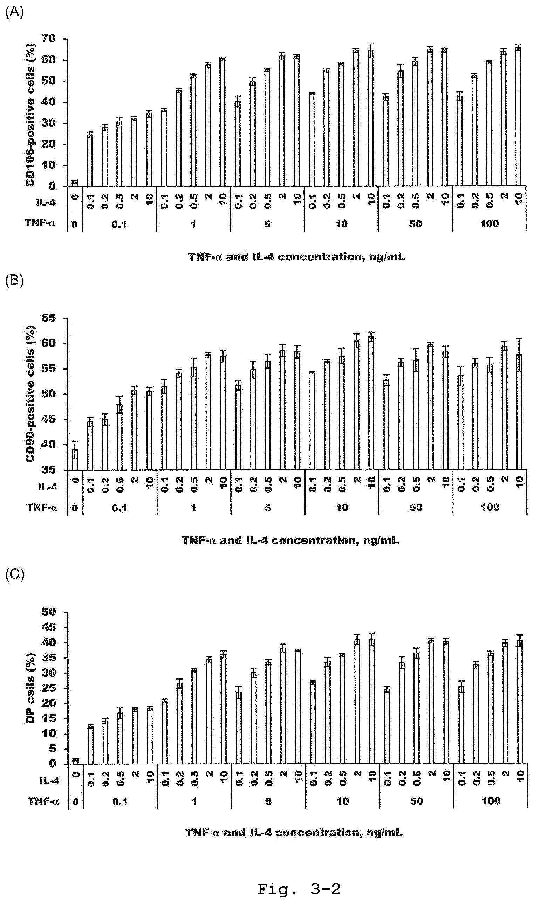

FIG. 3-2 shows graphs representing (A) the percentage of CD106-positive cells, (B) the percentage of CD90-positive cells, and (C) the percentage of double-positive (DP) cells in the presence of various concentration of added TNF-α and IL-4 (N = 3).

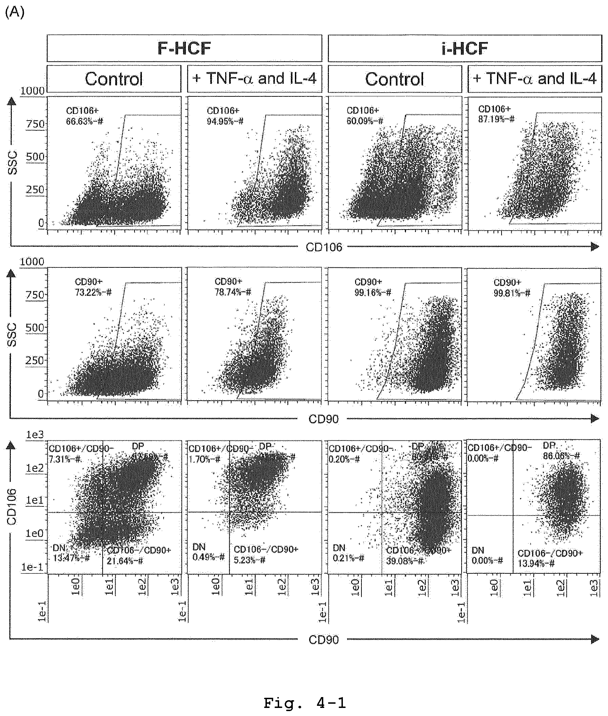

FIG. 4-1 shows: (A) shows the flow cytometry analysis of fetal cardiac fibroblasts (F-HCF) and iPS cardiomyocyte fraction-derived fibroblasts (i-HCF) with addition of TNF-α (50 ng/mL) and IL-4 (2 ng/mL). As controls were shown the flow cytometry analysis of F-HCF and i-HCF without addition of TNF-α (50 ng/mL) and IL-4 (2 ng/mL).

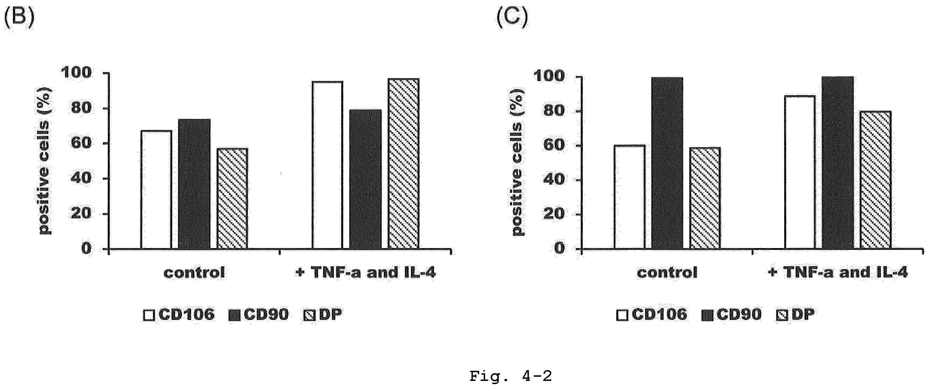

FIG. 4-2 shows: (B) the percentage (%) of CD106- and CD90-positive cells in F-HCFs with addition of TNF-α (50 ng/mL) and IL-4 (2 ng/mL) (N = 1). As controls were shown the percentage (%) of CD106- and CD90-positive cells in F-HCFs without addition of TNF-α (50 ng/mL) and IL-4 (2 ng/mL) (N=1). (C) shows the percentage (%) of CD106- and CD90-positive cells in i-HCFs with addition of TNF-α (50 ng/mL) and IL-4 (2 ng/mL) (N = 1). As controls were shown the percentage (%) of CD106- and CD90-positive cells in i-HCFs without addition of TNF-α (50 ng/mL) and IL-4 (2 ng/mL) (N=1).

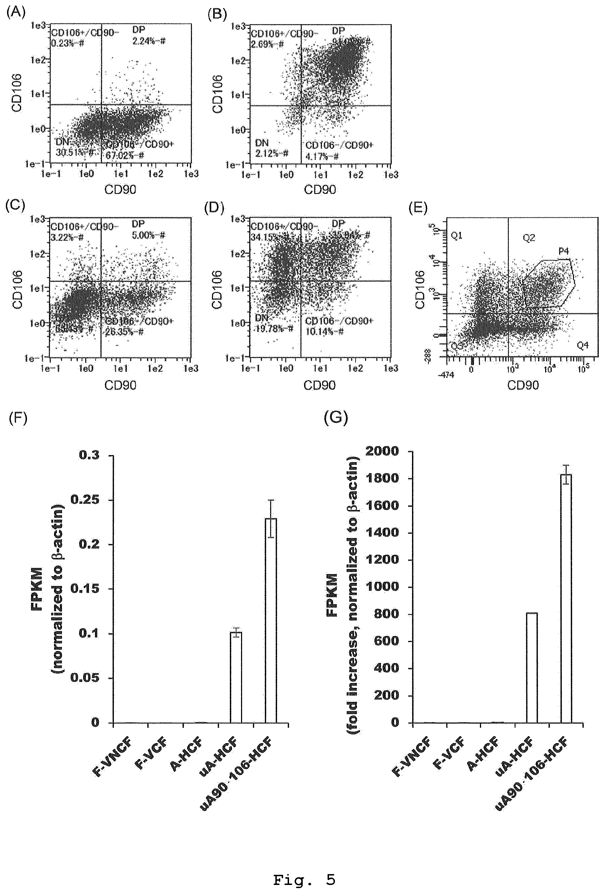

FIG. 5 shows: (A) shows the flow cytometry analysis of fetal CD106-negative cardiac fibroblasts (F-VNCF). (B) shows the flow cytometry analysis of fetal CD106-positive cardiac fibroblasts (F-VCF). (C) shows the flow cytometry analysis of adult cardiac fibroblasts (A-HCF). (D) shows the flow cytometry analysis of A-HCFs cultured with addition of TNF-α (50 ng/mL) and IL-4 (2 ng/mL) (uA-HCF). (E) shows the flow cytometry analysis of uA-HCF cell population positive for both CD106 and CD90 (uA90·106-HCF) collected with fluorescence-activated cell sorting (FACS). Only the cells in the enclosed gate (P4) in the figure are collected by FACS. (F) is a graph showing the expression level of the G-CSF gene in various fibroblasts (FPKM of G-CSF normalized to FPKM of β-actin) (N = 3 for F-VCF and F-VNCF; N = 2 for the others). (G) is a graph showing the expression level of the G-CSF gene in various fibroblasts (fold increase of FPKM of G-CSF normalized to FPKM of β-actin; the value of F-VCF is set at 1) (N = 3 for F-VCF and F-VNCF; N = 2 for the others).

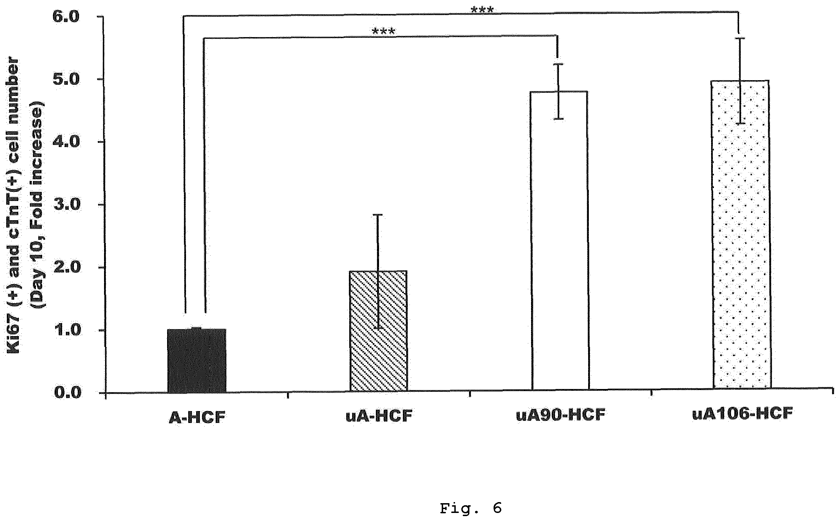

FIG. 6 is a graph showing the number of Ki67 and cardiac troponin T (cTnT)-double positive cells in iPS-derived cardiomyocytes (iPS-CM) co-cultured with various cardiac fibroblasts (Day 10, ***P < 0.01). uA90-HCF refers to a CD90-positive uA-HCF cell population after cell sorting using CD90 as a marker. uA106-HCF refers to a CD106-positive uA-HCF cell population after cell sorting using CD106 as a marker. The number of Ki67 and cTnT-double positive cells in iPS-CM co-cultured with A-HCF is set at 1 (N = 3).

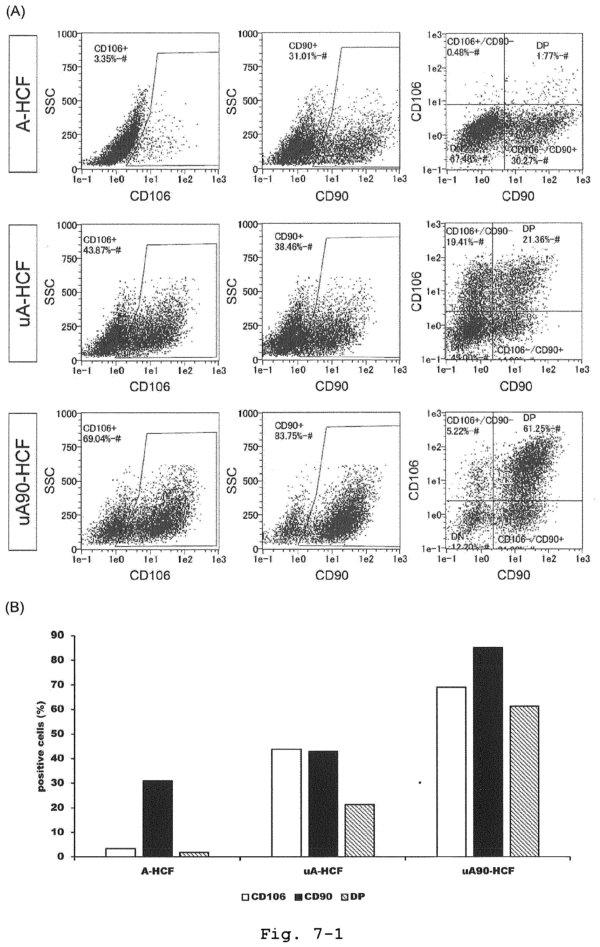

FIG. 7-1 shows: (A) shows the flow cytometry analysis of the fibroblasts administered to a chronic heart failure model rat. (B) shows graphs representing the percentage of CD106-positive cells, the percentage of CD90-positive cells, and the percentage of double-positive (DP) cells in the fibroblasts administered to a chronic heart failure model rat.

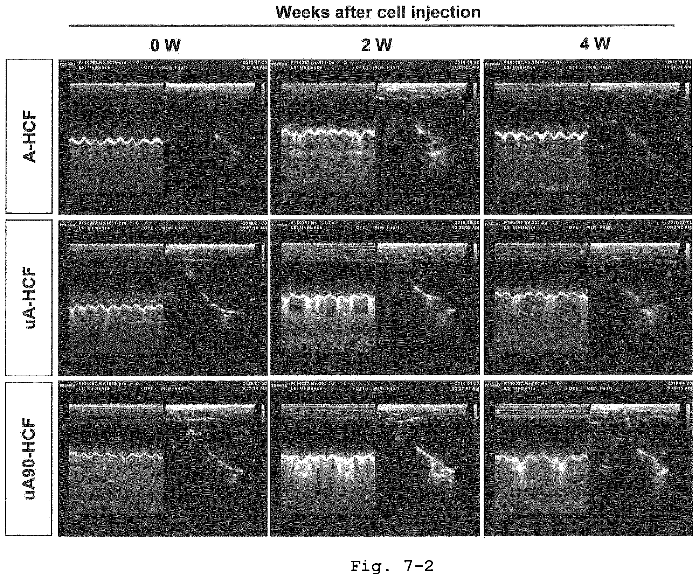

FIG. 7-2 shows echocardiographic images (M mode) of a rat heart (photograph substitute for Figure).

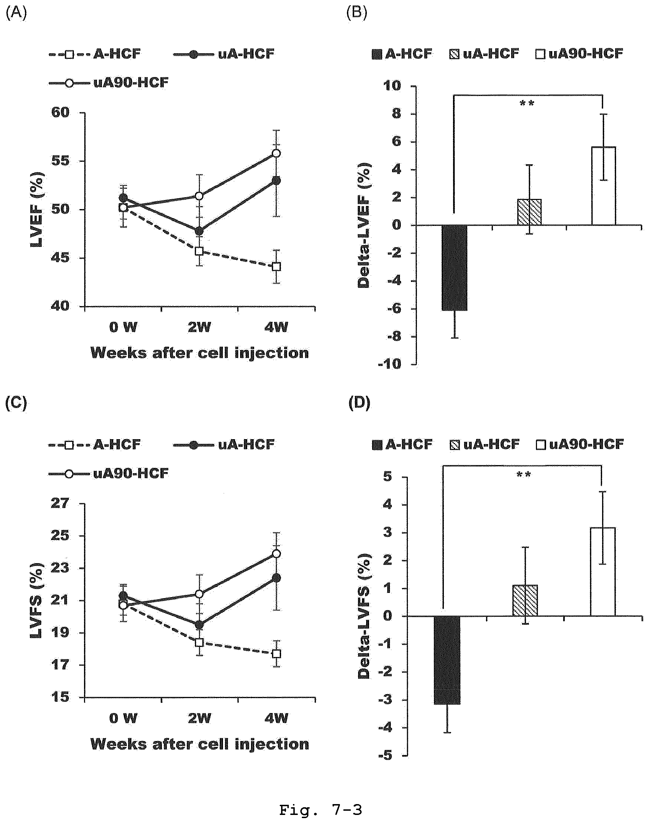

FIG. 7-3 shows: (A) is a graph showing the change in LVEF (%) of a rat heart in the presence of various cardiac fibroblasts. (B) is a graph showing % change in LVEF 4 weeks after injection of various cardiac fibroblasts (LVEF (4W) - LVEF (0W), **P < 0.05, N = 4 for A-HCF and uA-HCF administered groups; N = 3 for uA90-HCF administered group). (C) is a graph showing the change in LVFS (%) of a rat heart in the presence of various cardiac fibroblasts. (D) is a graph showing % change in LVFS 4 weeks after injection of various cardiac fibroblasts (LVFS (4W) - LVFS (0W), **P < 0.05, N = 4 for A-HCF and uA-HCF administered groups; N = 3 for uA90-HCF administered group).

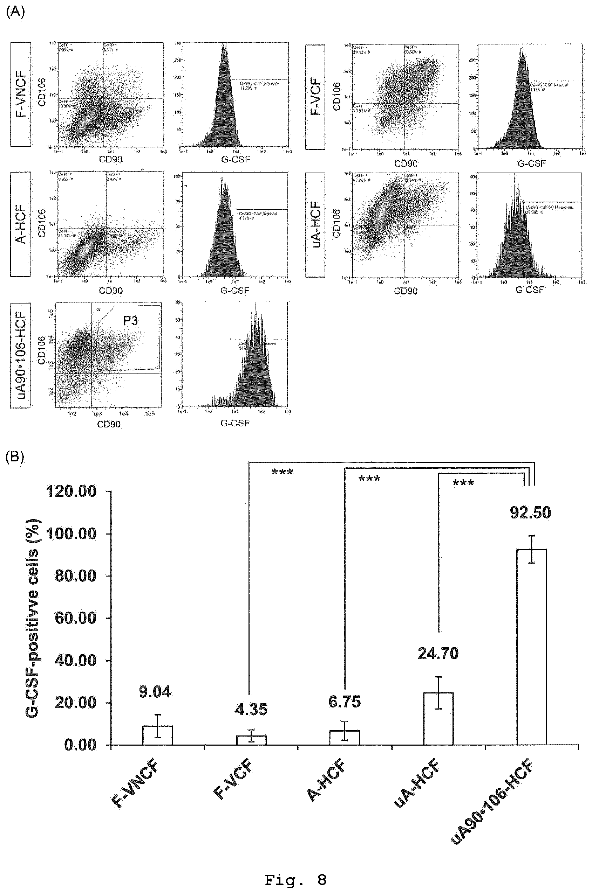

FIG. 8 shows: (A) shows the flow cytometry analysis of various fibroblasts. (B) is a graph showing the percentage (%) of G-CSF-positive cells in various fibroblasts (***P < 0.01, N = 4). The uA90·106-HCF is a uA-HCF cell population positive for both CD106 and CD90 collected with fluorescence-activated cell sorting (FACS). Only the cells in the enclosed gate (P3) in the figure are collected by FACS.

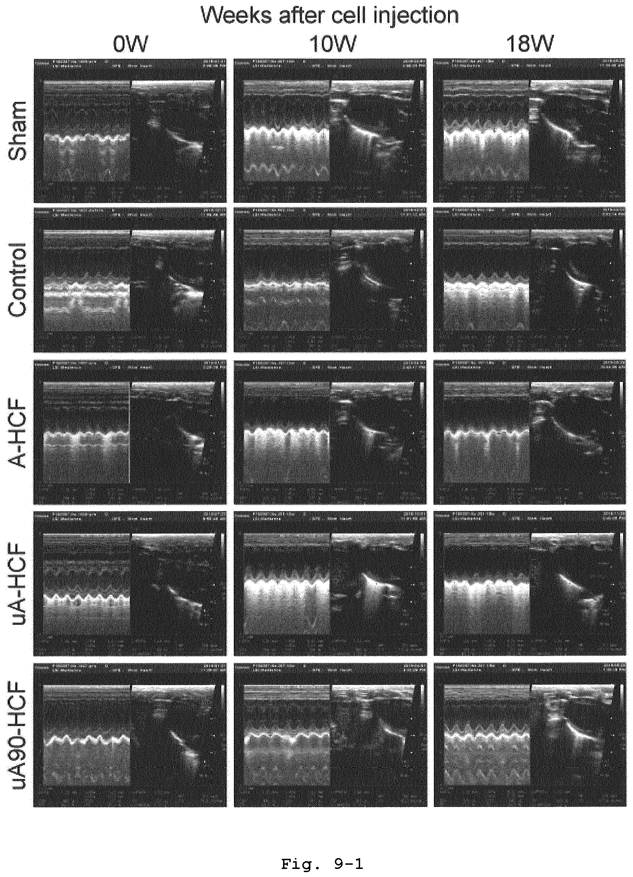

FIG. 9-1 shows echocardiographic images (M mode) from chronic heart failure model rats administered with various cardiac fibroblasts (photograph substitute for Figure).

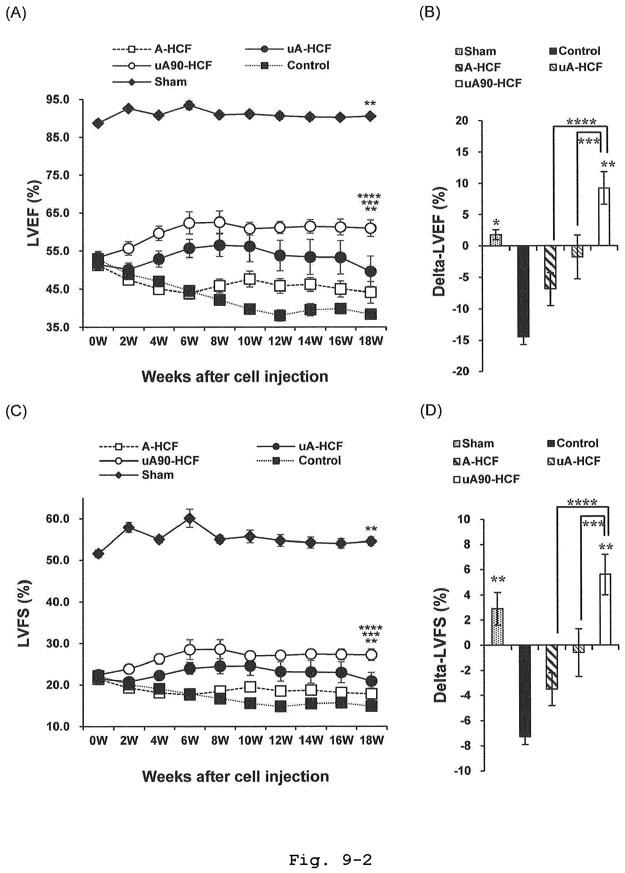

FIG. 9-2 shows: (A) is a graph showing the change in LVEF (%) from rat models of chronic heart failure administered with various cardiac fibroblasts (****P < 0.01 vs. A-HCF, ***P < 0.05 vs. uA-HCF, **P < 0.01 vs. Control (a group administered with only the medium (high-glucose DMEM + 10% newborn calf serum (NBCS))), N = 6 for Sham (a group receiving the same thoracotomy as Control and cell- administered group, but not receiving ischemia-reperfusion), N = 4 for Control, N = 7 for A-HCF administered group, N = 8 for uA-HCF administered group, N = 9 for uA90-HCF administered group). (B) is a graph showing % change in LVEF 18 weeks after injection of various cardiac fibroblasts (LVEF (18W) - LVEF (0W), ****P < 0.01 vs. A-HCF, ***P < 0.01 vs. uA-HCF, **P < 0.01 vs. Control, *P < 0.05 vs. Control). (C) is a graph showing the change in LVFS (%) from rat models of chronic heart failure in the presence of various cardiac fibroblasts (****P < 0.01 vs. A-HCF, ***P < 0.05 vs. uA-HCF, **P < 0.01 vs. Control). (D) is a graph showing % change in LVFS 18 weeks after injection of various cardiac fibroblasts (LVFS (18W) - LVFS (0W), ****P < 0.01 vs. A-HCF, ***P < 0.05 vs. uA-HCF, **P < 0.01 vs. Control).

### DETAILED DESCRIPTION OF THE INVENTION

The present invention will now be described in more detail below using specific embodiments. The present invention is, however, not limited to the illustrated specific embodiments.

In one embodiment of the present invention, the method of producing CD106-positive (also referred to as CD106⁺) and/or CD90-positive (also referred to as CD90⁺) fibroblasts comprise the step of culturing fibroblasts in the presence of at least one selected from the group consisting of TNF-α and IL-4 to increase the number of CD106-positive and/or CD90-positive fibroblasts. The culturing in the presence of at least one selected from the group consisting of TNF-α and IL-4 leads to production of a fibroblast population comprising an increased number of CD106⁺ and/or CD90⁺ fibroblasts.

As used herein, the term "enriching" means an operation of separating cells through which the ratio of the number of a specific cell to the total cell number is increased. It should be noted that as used herein the term "culturing" is not included in the term "enriching." As used herein, the term "isolating" means separating a certain component from a tissue, while the term "purifying" means separating a certain component from at least one or more other components.

As used herein, the term "positive" means that cells express a detectable level of a marker.

As used herein, the term "comprising" means it may optionally include an unspecified third component. The term "consisting of," as used herein, means that it does not essentially include any unspecified third component. The term "not essentially including," is intended not to preclude the inclusion of a third component contaminated during the production process in such an amount that it cannot be removed from technical point of view.

Fibroblasts are a type of interstitial cells that produce extracellular matrices, such as collagen. Fibroblasts are cell species that exists in and mainly constitutes connective tissues in living body. The fibroblasts may be positive for at least one selected from the group consisting of vimentin and DDR2 that are markers of interstitial cells. In some aspects, the fibroblasts are not positive for cardiac troponin and alpha-actinin that are markers specific to cardiomyocytes. In some aspects, the fibroblasts are not positive for VE-cadherin. For example, the fibroblasts are not vascular endothelial cells. For example, the fibroblasts are not vascular smooth muscle cells. The fibroblasts may or may not be myofibroblasts.

The fibroblasts may be derived from a cardiac tissue, including fibroblasts isolated from a cardiac tissue. The fibroblasts may be isolated from an epicardium or endocardium. The fibroblasts may be isolated from a fetal epicardium or endocardium. The fibroblasts may be isolated from an adult epicardium or endocardium. The fibroblasts may be positive for at least one selected from the group consisting of vimentin and DDR2 and have potential to produce collagen. In all aspects of the present invention, the fibroblasts may preferably be derived from a cardiac tissue (hereinafter may also referred to as "cardiac fibroblasts"), including fibroblasts isolated from an epicardium or endocardium.

In the embodiments of the present invention, any cells that differentiate into fibroblasts in living body may be used as source of fibroblasts.

In the preferred embodiments of the present invention, the fibroblasts used for transplantation to hearts may be derived from a cardiac tissue, an epicardium, or an endocardium.

In the embodiments of the present invention, the fibroblasts may be enriched, isolated, or purified.

The fibroblasts may be derived from any source, and may be used with being differentiated from pluripotent stem cells, such as embryonic stem cells (ES cells), induced pluripotent stem cells (iPS cells) or Muse cells, or adult (somatic) stem cells, such as mesenchymal stem cells. Alternatively, primary cells collected from an animal (including human) may be used, or established cells may be used. Preferably the fibroblasts may be derived from an epicardium or endocardium, or obtained from a human adult heart, but the present invention is not limited thereto.

While the fibroblasts have been discussed above, the same is true in all aspects, where the fibroblasts are derived from a cardiac tissue or the fibroblasts are isolated from an epicardium or endocardium. The present invention will be described in Examples below with reference to experiments using illustrative cardiac fibroblasts as fibroblasts.

CD106, which is also called VCAM-1 (VCAM1), is a protein known as a cell adhesion molecule expressed in, for example, vascular endothelial cells. CD90, which is also called Thy-1 (Thy1), is a heavily-glycosylated glycosylphosphatidylinositol (GPI)-linked molecule, and is expressed in various stromal cells, such as nerve tissues and connective tissues, but not in cardiomyocytes. Thus, CD90⁺ fibroblasts indicate that they do not include cardiomyocytes. The term "CD90⁺ fibroblasts do not include cardiomyocytes" here is a concept that inclusion of some cardiomyocytes is acceptable, and cardiomyocytes may be 5% or less, 4% or less, 3% or less, 2% or less, 1% or less, 0.5% or less, 0.1% or less, or 0.01% or less related to total cells.

The percentage of CD90⁺ fibroblasts in CD106⁺ fibroblasts (based on the cell number) may be 1% or more, 10% or more, 20% or more, 30% or more, 40% or more, 50% or more, 60% or more, 70% or more, 80% or more, 90% or more, 95% or more, 98% or more, or 100%.

The fibroblasts may be Connexin 43-positive (Connexin 43⁺) fibroblasts.

Connexin 43 is a transmembrane protein that is known to be expressed on the surface of blood vessels in association with atherosclerotic plaque, and to link neighboring cells as a gap junction protein of cardiomyocytes to propagate the electric excitation of the heart. The inventors believe that when the fibroblasts are positive for Connexin 43, it allows for communication of electrical signals in cardiac tissues and have improved therapeutic effect in application to heart diseases.

The percentage of Connexin43+ fibroblasts in CD106+ fibroblasts (based on the cell number) may be 1% or more, 10% or more, 20% or more, 30% or more, 40% or more, 50% or more, 60% or more, 70% or more, 80% or more, 90% or more, 95% or more, 98% or more, or 100%.

The method of contacting fibroblasts with at least one factor selected from the group consisting of TNF-α and IL-4 (hereinafter also referred to as simply "factor") is not particularly restricted. Typically, but without limitation, the factor is added to a medium containing fibroblasts. When being contacted with a plurality of the factors, fibroblasts may be contacted with each of them separately or simultaneously. As a method of simultaneously contacting fibroblasts with the factors, a plurality of factors may be first mixed and then added to fibroblasts.

In this embodiment, since fibroblasts can be cultured in a medium supplemented with the factor, the expression level(s) of CD106 and/or CD90 in fibroblasts can be maintained during the culturing. Thus, fibroblasts with high expression level(s) of CD106 and/or CD90 can be produced.

When these factors are added to a medium (mL), the amount of TNF-α to be added is not particularly limited, and is typically 0.1 ng/mL or more, or may be 0.5 ng/mL or more, 1 ng/mL or more, or 10 ng/mL or more. The upper limit is not particularly limited, and is typically 500 ng/mL or less, or may be 100 ng/mL or less.

The amount of IL-4 to be added is not particularly limited, and is typically 0.1 ng/mL or more, or may be 0.5 ng/mL or more, or 1 ng/mL or more. The upper limit is not particularly limited, and is typically 10 ng/mL or less, or may be 5 ng/mL or less or 1 ng/mL or less.

When both of the factors are added, the ratio (by wight) of TNF-α to IL-4 (TNF-α: IL-4) to be added is typically 10000:1 to 1:1, or may be 50000:1 to 10:1. Alternatively, the ratio is 1:1 to 1:10000, or may be 1:10 to 1:50000.

After contact with these factors, the fibroblasts can be further cultured to express CD106⁺ and/or CD90⁺ fibroblasts.

Fibroblasts may be cultured by any known cell culture technique, without particular limitation, under conditions that can express CD106⁺ and/or CD90⁺ or are suitable for the culture.

The medium for use in the culture can be determined as appropriate depending on, for example, the type of the cell to be cultured. Examples of the culture medium that can be used include DMEM, α-MEM, RPMI-1640, and HFDM-1(+). The culture medium may be supplemented with such as nutritive substances such as FCS and FBS, growth factors, cytokines, and antibiotics. The cells may be further cultured in a culture medium containing TNF-α and/or IL-4.

The culture period (the number of days) can be determined as appropriate according to the purpose, for example, to achieve the desired number of cells and/or impart the desired function. Examples of the period include one day, two days, three days, four days, five days, six days, seven days, eight days, nine days, ten days, two weeks, one month, two months, three months, and six months.

The culture temperature can be determined as appropriate according to the type of the cell to be cultured, and may be, for example, 10°C or higher, 15°C or higher, 20°C or higher, 25°C or higher, or 30°C or higher, and may be, for example, 60°C or lower, 55°C or lower, 50°C or lower, 45°C or lower, or 40°C or lower.

After being cultured, the fibroblasts may be collected in a collection step. In the collection step, cells may be collected by detaching the cells with proteases such as trypsin or with temperature changes using a temperature-responsive culture dish. Alternatively, cells may be collected or enriched using an anti-CD90 antibody and/or anti-CD106 antibody in an automated magnetic cell sorting system (e.g., autoMACS), a magnetic cell sorting system (e.g., MACS), a closed magnetic cell sorting system (e.g., Prodigy), or a cell sorter (e.g., FACS). A drug resistance gene may be introduced into the downstream of the promoter(s) of the gene(s) encoding CD90 and/or CD106 protein(s) and the cells may be selected with the drug.

The production method in the present embodiment may comprise a step of sorting out CD90⁺ fibroblasts using an anti-CD90 antibody, a step of sorting out CD106⁺ fibroblasts using an anti-CD106 antibody, or both of the two steps. The sorting step may be performed at any timing. The timing may be before the contact with the factors described above; after the contact with the factors and before the culture; or after the culture.

Any known anti-CD90 and anti-CD 106 antibodies can be used, including commercially available products. Sorting using an anti-CD90 antibody and/or anti-CD106 antibody can lead to increase of the percentage of CD106⁺ and/or CD90⁺ fibroblasts in fibroblasts and provide CD106⁺ and/or CD90⁺ fibroblasts that are highly effective in treatment of heart diseases.

The method of producing CD106⁺ and/or CD90⁺ fibroblasts in the present embodiment provide fibroblasts with increased expression of CD106 and/or CD90, which constitute a fibroblast population in another embodiment of the present invention. In particular, a cell population of fibroblasts comprising CD106⁺ and/or CD90⁺ adult fibroblasts shows increased expression of CD106 and/or CD90 due to stimulation with TNF-α and/or IL-4 and can be suitably used as a pharmaceutical composition.

The percentage of CD106⁺ fibroblasts in the total fibroblasts in the cell population may be, but not particularly limited to, 3.36% or more, 5% or more, 10% or more, 20% or more, 30% or more, 40% or more, 50% or more, 60% or more, 70% or more, 80% or more, 90% or more, or 95% or more based on the cell number.

The percentage of CD90⁺ fibroblasts in the total fibroblasts in the cell population may be, but not particularly limited to, 5% or more, 10% or more, 20% or more, 30% or more, 40% or more, 50% or more, 60% or more, 70% or more, 80% or more, 90% or more, or 95% or more based on the cell number.

The percentage of CD106⁺ and CD90⁺ fibroblasts in the total fibroblasts in the cell population may be, but not particularly limited to, 5% or more, 10% or more, 20% or more, 30% or more, 40% or more, 50% or more, 60% or more, 70% or more, 80% or more, 90% or more, or 95% or more based on the cell number.

The present inventors have also found that G-CSF-positive fibroblasts can be produced by contacting fibroblasts with at least one factor selected from the group consisting of TNF-α and IL-4.

G-CSF has been reported to reduce cardiomyocyte death associated with heart failure and prevent progression of myocardial remodeling (Harada, M. et al., G-CSF prevents cardiac remodeling after myocardial infarction by activating the Jak-Stat pathway in cardiomyocytes. Nat.Med.11, 305-311 (2005)). It has also been revealed that G-CSF increases cell growth of cardiomyocytes (Shimoji, K. et al., G-CSF Promotes the Proliferation of Developing Cardiomyocytes In Vivo and in Derivation from ESCs and iPSCs. Cell Stem Cell 6, 227-237 (2010)).

Accordingly, in another embodiment of the present invention, a method of producing G-CSF-positive fibroblasts comprise the steps of contacting fibroblasts with at least one selected from the group consisting of TNF-α and IL-4; and culturing the fibroblasts after the contact under such conditions that the expression of G-CSF is increased.

The description of the method of producing CD106⁺ and/or CD90⁺ fibroblasts can be referred to for the contacting step, culturing step, etc. in the present embodiment. The created G-CSF-positive fibroblasts may be enriched using an anti-CD90 antibody and/or anti-CD106 antibody in an automated magnetic cell sorting system (e.g., autoMACS), a magnetic cell sorting system (e.g., MACS), a closed magnetic cell sorting system (e.g., Prodigy), or a cell sorter (e.g., FACS). A drug resistance gene may be introduced into the downstream of the promoter of the gene encoding a G-CSF protein and the cells may be selected with the drug.

The method of creating G-CSF-positive fibroblasts in the present embodiment provides G-CSF-positive fibroblasts. In other embodiments of the present invention, a fibroblast population comprising the G-CSF-positive fibroblasts is also provided. The fibroblast population can be suitably used as a pharmaceutical composition. In addition, the cell population comprising G-CSF-positive fibroblasts can be applied in a method of treating heart diseases comprising the step of injecting it into a necrosed cardiac tissue region or a peripheral region thereof and/or into a coronary artery.

The percentage of G-CSF-positive fibroblasts in the total fibroblasts in the cell population comprising fibroblasts may be, but not particularly limited to, 1% or more, 5% or more, 6.75% or more, 10% or more, 20% or more, 30% or more, 40% or more, 50% or more, 60% or more, 70% or more, 80% or more, 90% or more, 95% or more based on the cell number.

For the expression level of the gene encoding the G-CSF protein in fibroblasts, the FPKM with normalization to beta-actin may be 0.01 or more, 0.02 or more, 0.03 or more, 0.04 or more, 0.05 or more, or 0.1 or more.

In addition, the expression level of the gene encoding the G-CSF protein in fibroblasts may be 1.1-fold or more, 2-fold or more, 5-fold or more, 10-fold or more, 20-fold or more, 50-fold or more, 100-fold or more, 200-fold or more, of 500-fold or more as compared with the expression level in fibroblasts in nature (living body).

Further, the G-CSF-positive fibroblasts may be positive for CD106 and/or CD90, and the percentage of the fibroblasts (based on the cell number) may be 1% or more, 10% or more, 20% or more, 30% or more, 40% or more, 50% or more, 60% or more, 70% or more, 80% or more, 90% or more, 95% or more, 98% or more, or 100%.

The percentage of the G-CSF-positive fibroblasts in the fibroblasts positive for CD106 and/or CD90 in the cell population comprising fibroblasts (based on the cell number) may be 1% or more, 3% or more, 5% or more, 10% or more, 25% or more, or 50% or more.

In particular, the G-CSF-positive, CD106-positive, and CD90-positive fibroblasts in the cell population comprising fibroblasts may be 1% or more, 5% or more, 7% or more, 10% or more, 20% or more, 30% or more, 40% or more, 50% or more, 60% or more, 70% or more, 80% or more, 90% or more, 95% or more, 98% or more, or 100%, based on the cell number.

A cell population of fibroblasts as a pharmaceutical composition may contain other components that are physiologically acceptable in a pharmaceutical composition in addition to the cell population of fibroblasts.

The pharmaceutical composition in the present embodiment can be applied to patients with heart diseases to improve the functions of their heart. The heart diseases in the present embodiment include diseases caused by, for example, disorders in, deficiencies in, and dysfunctions of cardiac tissues, including, but not limited to, heart failure, ischemic heart diseases, myocardial infarction, cardiomyopathy, myocarditis, hypertrophic cardiomyopathy, and dilated cardiomyopathy. Thus, the present invention provides a pharmaceutical composition comprising CD106⁺ and/or CD90⁺ fibroblasts or G-CSF⁺ fibroblasts, for use in improvement of cardiac functions, for example, for use in growth of cardiac muscle in vivo and/or improvement of the cardiac ejection fraction. The present invention also provides a pharmaceutical composition comprising CD106⁺ and/or CD90⁺ fibroblasts or G-CSF⁺ fibroblasts, for use in prevention of progression of fibrosis in cardiac tissues.

The CD106⁺ and/or CD90⁺ fibroblasts or G-CSF⁺ fibroblasts secrete cytokines and the like to control the inflammatory response for maintenance of organ homeostasis. The secreted cytokines, chemokines and the like may lead to formation of suitable microenvironment for regeneration of cardiac muscle tissues, allowing for growth of cardiomyocytes and adjustment of beating of cardiomyocytes. The CD106⁺ and/or CD90⁺ fibroblasts or G-CSF⁺ fibroblasts may also prevent progression of fibrosis.

An illustrative method of administering the pharmaceutical composition to a patient with heart disease is injection.

When the pharmaceutical composition is used as an injectable (injectable composition), the injectable may comprise other cells and other components in addition to CD106⁺ and/or CD90⁺ fibroblasts or G-CSF⁺ fibroblasts. When other cells are contained, the percentage of CD106⁺ and/or CD90⁺ fibroblasts or G-CSF⁺ fibroblasts in the total fibroblasts contained in the injectable may be 0.03% or more, 0.1% or more, 1% or more, 2% or more, 4% or more, 5% or more, 6.75% or more, 10% or more, 20% or more, 30% or more, 40% or more, 50% or more, 60% or more, 70% or more, 80% or more, 90% or more, 95% or more, 98% or more, or 99% or more, based on the cell number.

In the case of an injectable, the number of CD106⁺ and/or CD90⁺, and G-CSF⁺ fibroblasts contained in the injectable may be 1 × 10⁶ cells or more, 5 × 10⁶ cells or more, or 1 × 10⁷ cells or more.

The CD106⁺ and/or CD90⁺ fibroblasts or G-CSF⁺ fibroblasts contained in the injectable composition may be co-cultured with other cells, for example, cardiomyocytes.

The injectable may contain other components that is physiologically acceptable as those contained in an injectable. Examples of such other components include physiologic saline, phosphate buffered saline (PBS), cell preservation solution, cell culture medium, hydrogel, extracellular matrix, and cryopreservation solution.

The injectable allows adult fibroblasts to be injected into a cardiac tissue, for example, a necrotic cardiac tissue region or a peripheral region thereof and/or into a coronary artery or into a vein, an artery, a lymph node, or a lymphatic vessel to treat heart diseases. As the source of the adult fibroblasts, the heart disease patient's own tissues may be used for autologous transplantation.

The fibroblast population may be used as a scaffolding for culture of other cells, or may be used for formation of an organ or tissue, for example, for organization of a planar or three-dimensional cellular tissue. The fibroblast population may be co-cultured with other cells such as cardiomyocytes to obtain a planar or three-dimensional tissue, but the fibroblast population effectively functions as a planar or three-dimensional tissue without the co-culture. Examples of the planar or three-dimensional tissue include, but not limited to, cell sheet, cell fiber, tissue formed with a 3D printer.

Such a planar or three-dimensional tissue can be applied on a necrotic cardiac tissue region or replaced with a necrotic cardiac tissue as an artificial organ to treat heart diseases.

### EXAMPLES

The present invention will be described in more detail below with reference to Examples, which do not limit the scope of the present invention.

### <Animals, Cells, and Reagents>

All animal experimentation protocols performed in this study were approved by the ethics committee of LSI Medience Corporation (Tokyo, Japan). All animals received refinement alternatives.

Cells were purchased from the following manufacturers: adult human cardiac fibroblast (Lonza, Basel, Switzerland); fetal human cardiac fibroblast (Cell Applications, San Diego, CA); and human iPS cell-derived cardiomyocyte (iPS-CM, Myoridge, Kyoto, Japan). Human iPS cell-derived cardiac fibroblasts were isolated from iPS-CMs based on the difference in the adhesiveness to a substrate surface.

The following antibodies were used in the immunofluorescent staining and flow cytometry analysis: BV421, mouse anti-human CD106 antibody (BD Biosciences, San Jose, CA); BV421, mouse IgG1; kappa isotype control (BD Biosciences); human CD90-PE (Miltenyi Biotec, Bergisch Gladbach, Germany); REA control (S)-PE (Miltenyi Biotec); mouse anti-cardiac troponin T (cTnT) monoclonal antibody (Thermo Scientific, Waltham, MA); rabbit anti-Ki67 polyclonal antibody (Abcam, Cambridge, UK); Hoechst 33258 solution (Dojindo Laboratories, Kumamoto, Japan); Ms mAb to G-CSF (Abcam); and APC Goat antimouse IgG (BioLegend, San Diego, CA). For analysis of proteins localized in cytoplasm, cells were subjected to cell membrane permeabilization using 0.1% Triton-X (Sigma Aldrich, St. Louis, MO) for 30 minutes (at room temperature) followed by immunofluorescent staining for fluorescence microscopy. For flow cytometry analysis, each type of fibroblasts was subjected to cell membrane permeabilization using 0.1% saponin (Nacalai Tesque, Kyoto, Japan) for 15 minutes (at room temperature) followed by immunofluorescent staining.

### <Cell Sorting>

F-VNCF (CD106-negative fetal cardiac fibroblasts), F-VCF (CD106-positive fetal cardiac fibroblasts), and uA106-HCF (CD106-positive adult cardiac fibroblasts obtained by adding TNF-α and IL-4 to adult cardiac fibroblasts to increase the percentage of cells positive for CD90 and CD106 and then subjecting them to cell sorting using CD106 as a marker) were subjected to primary immunostaining with human CD106 (VCAM-1)-biotin (Miltenyi Biotec), followed by secondary immunostaining with anti-biotin microbeads (Miltenyi Biotec). uA90-HCF (CD90-positive adult cardiac fibroblasts obtained by adding TNF-α and IL-4 to adult cardiac fibroblasts to increase the percentage of cells positive for CD90 and CD106 and then subjecting them to cell sorting using CD90 as a marker) were subjected to primary immunostaining with human CD90 -biotin (Miltenyi Biotec), followed by secondary immunostaining with anti-biotin microbeads (Miltenyi Biotec). The stained cells were subjected to autoMACS (Miltenyi Biotec) to collect CD106- and CD90-positive cells.

Adult cardiac fibroblasts (uA90·106-HCF) with a high percentage of CD90-and CD106-positive cells were collected by treating adult cardiac fibroblasts with TNF-α and IL-4 to increase the percentage of CD90- and CD106-positive cells; immunostaining the cells with BV421, a mouse anti-human CD106 antibody (BD Biosciences, San Jose, CA), BV421, mouse IgG1; kappa isotype control (BD Biosciences), human CD90-PE (Miltenyi Biotec, Bergisch Gladbach, Germany), or REA control (S)-PE (Miltenyi Biotec); and subjecting the cells to cell sorting with BD FACS ARIAIII (BD Biosciences).

### <Flow Cytometry>

Cells after the immunofluorescent staining were prepared into a concentration of 1.0 × 10⁶ cells/trial and analyzed using a flow cytometer (MACSQuant Analyzer 10, Miltenyi Biotec). After recognition of the cell region with FSC-A and SSC-A, the percentages of cells positive for various marker proteins (%, in terms of the number of cells) were evaluated.

### <RNA Extraction and mRNA Sequencing>

RNA was collected from each type of fibroblasts using Qiagen RNeasy Mini Kit (QIAGEN, Valencia, CA). Adult cardiac fibroblasts (uA90·106-HCF) with a high percentage of CD90- and CD106-positive cells were collected by treating adult cardiac fibroblasts with TNF-α and IL-4 to increase the percentage of CD90- and CD106-positive cells; and then subjecting the cells to cell sorting with BD FACS ARIAIII (BD Biosciences). The collected RNA was analyzed for its concentration and purity using Agilent 2100 Bioanalyzer (Agilent Technologies, Palo Alto, CA) and NanoDrop One (Thermo Fisher Scientific). One microgram of RNA with an RIN value of 7 or more was used for library preparation. Library preparation for next generation sequencer was performed according to the protocol from the manufacturer of NEBNext Ultra RNA Library Prep Kit for Illumina.

Poly(A) mRNA was isolated using NEBNext Poly(A) mRNA Magnetic Isolation Module (NEB) and Ribo-Zer rRNA removal Kit (Illumina). mRNA was fragmented and primed using NEBNext First Strand Synthesis Reaction Buffer and NEBNext Random Primers.

A first strand cDNA was synthesized using ProtoScript II Reverse Transcriptase. A second strand cDNA was synthesized using Second Strand Synthesis Enzyme Mix. After purification using AxyPrep Mag PCR Clean-up (Axygen, UnionCity, CA), the double-stranded cDNA was subjected to repair of the ends, addition of dA to the end of the amplified products, and then treatment with End Prep Enzyme Mix for TA cloning. Regarding the size of Adaptor-ligated DNA, up to 360 bp of fragments were selected using AxyPrep Mag PCR Clean-up (Axygen). Samples were amplified for 11 cycles by PCR using P5 and P7 primers. The PCR products were cleaned up using AxyPrep Mag PCR Clean-up (Axygen), and evaluated using Agilent 2100 Bioanalyzer (Agilent Technologies). The PCR products were quantified using Qubit 2.0 Fluorometer (Invitrogen, Carlsbad, CA).

Sequencing was performed on Illumina HiSeq (Illumina) using a 2 × 150bp paired-end (PE) configuration. Image analysis and base calling were performed using HiSeq Control Software (HCS) + OLB + GAPipeline-1.6 (Illumina). Sequencing was performed on GENEWIZ (SouthPlainfield, NJ).

### <Differential Expression Analysis>

Differential expression analysis was performed using DESeq Bioconductor package. The false discovery rate (FDR) was corrected using the Benjamini and Hochberg method. P-value < 0.05 was considered significant.

### <Co-culture of Cardiomyocytes and Fibroblasts>

A 384-well plate (Thermo Fisher Scientific) was coated with 100 µL/well of Matrigel Basement Membrane Matrix (Corning, Corning, NY) diluted 1:30 with high-glucose DMEM at 37°C for 1 hour before cell seeding. iPS-CMs and fibroblasts were co-cultured in DMEM + 10% newborn calf serum (NBCS) at a ratio of 8:2 (cardiomyocytes: fibroblasts = 16,000: 4,000 (cells/well)).

After the start of co-culture, cells were fixed with 4% paraformaldehyde and fluorescently immunostained on Day 10. The stained samples were analyzed with IN Cell Analyzer 2200 (GE Healthcare, Buckinghamshire, UK) and IN Cell Developer Toolbox 1.92 (GE Healthcare).

### <Preparation and Echocardiography of Chronic Heart Failure Model Rat>

Nude rats (F344/NJcl-*rnu*/*rnu*, 8-weeks, male) were purchased from CLEA Japan (Tokyo, Japan). After 1 week of acclimation, each animal was subjected to inhalation anesthesia with 2% isoflurane (anesthetic adjuvant; laughing gas:oxygen=7:3) using an inhalation anesthesia apparatus for experimental animals (Soft Lander (Shin-ei Industries, Inc., Saitama, Japan)), and then the hair was clipped. Immediately thereafter, tracheal intubation was carried out, and 0.5 to 2% isoflurane (anesthetic adjuvant; laughing gas: oxygen=7:3) inhalation anesthesia gas was directly introduced into the respirator to maintain anesthesia. Under artificial respiratory management, the animal was fixed in a supine position, and thoracotomy was performed by longitudinally cutting, at costal cartilage, two or three ribs between the left third rib and fifth rib. Using a retractor, the operative field was expanded, and the pericardial membrane was detached to expose the heart. The left atrium was lifted up, and a thread was passed at a depth of about 2 mm through a length of 4 to 5 mm in the left ventricle using an atraumatic weakly curved round needle for blood vessels (6-0: Nescosuture). Both ends of the thread were combined together, and a snare prepared with a polyethylene tube was passed therethrough. The thread was tightened using an artery clamp (snare method) to cause coronary artery ischemia for 30 minutes. Thereafter, reperfusion was carried out.. After the conditions became stable, the absence of bleeding was confirmed, and chest drainage was carried out, followed by suture of the muscle layer and the skin. For the skin, subcuticular suture was performed. When normal suture was performed, suture removal was carried out depending on the postoperative conditions monitored. One week after the preparation of the model, that is, one day before the administration of the cells, echocardiographic measurement was carried out using an ultrasonic imaging diagnostic apparatus (Xario SSA-660A, Toshiba Medical Systems, Tochigi, Japan). Individuals having a left ventricular ejection fraction (LVEF=(LVIDd3-LVIDs3)/LVIDd3) of not more than 55% were regarded as a chronic heart failure model, and subjected to fibroblasts administration experiment.

### <Administration of Fibroblasts to Chronic Heart Failure Model Rat>

On the day of the administration test, cryopreserved fibroblasts were thawed, diluted with high-glucose DMEM + 10% NBCS, and 2.0 × 10⁶ cells /50 µL in terms of the live cell number, of the cell suspension was administered to each individual. The tests were performed at N = 4 for A-HCF and uA-HCF administered groups, and at N = 3 for uA90-HCF administered group. While anesthesia was maintained by the same method as in the preparation of the model, the total amount, 50 µL, of the cell suspension was administered to the animals using a catheter with a 30-G needle dividedly at two positions in the infarct region under artificial respiratory management. After the conditions became stable, the absence of leakage of the administered liquid or bleeding was confirmed, and chest drainage was carried out, followed by suture of the muscle layer and the skin. For the skin, subcuticular suture was performed. When normal suture was performed, suture removal was carried out depending on the postoperative conditions monitored.

### <Evaluation of Cardiac Functions of Chronic Heart Failure Model Rat by Echocardiography>

The chronic heart failure model rat to which fibroblasts were administered was followed up while echocardiographic measurement was carried out using an ultrasonic imaging diagnostic apparatus (Xario SSA-660A) every two weeks. Specifically, hair on the chest of the animal was clipped with a hair clipper, and a probe was placed on the chest to measure the left ventricular ejection fraction (LVEF = (LVIDd3 - LVIDs3/LVIDd3) and the left ventricular fractional shortening (LVFS = (LVIDd - LVIDs) × 100/LVIDd) by M-mode. The cardiac function values are expressed to one decimal place (by rounding to one decimal place)

### <Statistical Analysis>

The evaluation of cardiac function is represented as mean ± SE (standard error). Other data is represented as mean ± SD (standard deviation). Significant difference between two groups was calculated by student's t-test. Variation difference among three or more groups was calculated by one-way analysis of variance. Subsequently, significant difference among the three groups was calculated by Tukey-Kramer Multiple Comparison Test. Significance of difference was assumed when the p-value was smaller than 0.05. All statistics were calculated using R software.

The results of the performed experiments will be shown below.

### <Increase in Expression Levels of CD106 and CD90 in Cardiac Fibroblasts by TNF-α and IL-4>

Adult cardiac fibroblasts (A-HCF) were treated with TNF-α at various concentrations, cultured in HFDM-1 (+) medium for 3 days, and then subjected to evaluation of the percentage of CD106-positive cells (%) and the percentage of CD90-positive cells (%) with a flow cytometer. The results are shown in FIG. 1.

In addition, adult cardiac fibroblasts (A-HCF) were treated with IL-4 at various concentrations, cultured in HFDM-1 (+) medium for 3 days, and then subjected to evaluation of the percentage (%) of CD106-positive cells and the percentage (%) of CD90-positive cells with a flow cytometer. The results are shown in FIG. 2.

As can be seen from the results, addition of TNF-α or IL-4 increased the percentage (%) of CD106-positive cells and the percentage (%) of CD90-positive cells.

Next, adult cardiac fibroblasts (A-HCF) were treated with a combination of two agents, TNF-α and IL-4, cultured in HFDM-1 (+) medium for 3 days, and then subjected to evaluation of the percentage of CD106-positive cells (%) and the percentage of CD90-positive cells (%) with a flow cytometer. The results are shown in FIGS. 3-1 and 3-2. As can be seen from FIG. 3-1, addition of a combination of two agents, TNF-α and IL-4, shown in B significantly increased the percentage (%) of CD106-positive cells and the percentage (%) of CD90-positive cells, in comparison with control (untreated) shown in A.

Further, fetal cardiac fibroblasts (F-HCF) and iPS cell-derived cardiac fibroblasts (i-HCF) were treated with a combination of two agents, TNF-α (50 ng/mL) and IL-4 (2 ng/mL), cultured in HFDM-1 (+) medium for 3 days, and then subjected to evaluation of the percentage of CD106-positive cells (%) and the percentage of CD90-positive cells (%) with a flow cytometer. The results are shown in FIGS. 4-1 and 4-2.

As can be seen from FIGS. 4-1 and 4-2, even when cardiac fibroblasts were derived from different sources, addition of a combination of two agents, TNF-α and IL-4, significantly increased the percentage (%) of CD106-positive cells and the percentage (%) of CD90-positive cells.

Next, differential gene expression analyses of the following cells were performed: A-HCF; fibroblasts (uA-HCF) with the percentage of CD106- and CD90-positive cells being increased by culturing A-HCF for 3 days with HFDM-1 (+) medium supplemented with a combination of two agents, TNF-α (50 ng/mL) and IL-4 (2 ng/mL); and fibroblasts (uA90·106-HCF) obtained by cell sorting for only CD106- and CD90-double positive cells from uA-HCFs, using as controls fibroblasts (F-VCF) with the percentage of CD106- and CD90-positive cells being significantly increased and fibroblasts (F-VNCF) with the percentage of CD106-positive cells being significantly decreased by cell sorting of F-HCF with an anti-CD 106 antibody. The results are shown in FIG. 5. It was demonstrated from FIG. 5 that the different types of cardiac fibroblasts showed significantly differential expression levels of the gene encoding the granulocyte colony-stimulating factor (G-CSF) protein. It was demonstrated that the expression levels of the G-CSF gene in F-VNCF, F-VCF, and A-HCF (FPKM of G-CSF normalized to FPKM of β-actin) were 0.000178, 0.000125 and 0.000553, respectively, while the expression level was 0.101496 for uA-HCF, and 0.229072 for uA90·106-HCF. It was demonstrated that when the value of F-VCF was set as 1, the expression levels of the G-CSF gene in F-VNCF and A-HCF (fold increase of FPKM of G-CSF normalized to FPKM of β-actin) were 1.42 and 4.41, respectively, while the expression level was 810.21 for uA-HCF, and 1828.61 for uA90·106-HCF.

Thus, it was demonstrated that addition of a combination of two agents, TNF-α and IL-4, to A-HCF successfully increased the percentage of CD106- and CD90-positive cells, thereby significantly increasing the expression level of G-CSF. It was also demonstrated that cell sorting of uA-HCFs successfully increased the percentage of CD90- and CD106-positive cells, thereby further increasing the expression level of G-CSF. Preceding studies have reported that G-CSF reduces cardiomyocyte death associated with heart failure and prevents the progression of myocardial remodeling, and also have revealed that G-CSF promote the cell growth of cardiomyocytes. These results suggest that uA-HCF, uA90-HCF, uA106-HCF, and uA90·106-HCF with the expression level of G-CSF being artificially increased by TNF-α and IL-4 exhibit high cell growth ability of cardiomyocytes and high therapeutic effect on heart failure.

### <Growth Effect of Human iPS Cell-derived Cardiomyocytes by Cardiac Fibroblasts with Increased Expression Levels of CD106 and CD90>

Cardiac fibroblasts with increased percentage of CD90- and CD106-positive cells were prepared as described in Table 1, by addition of a combination of two agents, TNF-α (50 ng/mL) and IL-4 (2 ng/mL) and culture in HFDM-1(+) medium for 3 days. In addition, cardiac fibroblasts cultured without addition of TNF-α and IL-4 were prepared as a control.

The prepared cardiac fibroblasts with increased percentage of CD90- and CD106-positive cells were subjected to cell sorting using an anti-CD90 antibody or an anti-CD 106 antibody, so that CD90- and CD106-double positive (DP) fibroblasts were successfully and efficiently collected.

**Table 1**

| + TNF-α and IL-4 | cell sorting | abbreviation |
|---|---|---|
| - | - | A-HCF |
| + 50 ng/mL of TNF-α + 2 ng/mL of IL-4 | - | uA-HCF |
| | CD106+ | uA106-HCF |
| | CD90+ | uA90-HCF |

Next, each type of the cardiac fibroblasts shown in Table 1 and iPS-derived cardiomyocytes (iPS-CMs) were co-cultured, for evaluation of the growth effect of cardiac muscles by cardiac fibroblasts. For iPS-CMs, cells that was 92.22% positive for cardiac troponin T (cTnT), a marker specific for cardiomyocyte were used. The fibroblasts and iPS-CMs were co-cultured for 10 days thereafter Ki67- and cardiac troponin T (cTnT)-double positive cardiomyocytes in a proliferative state were counted. The results are shown in FIG. 6.

FIG. 6 showed that co-culture of uA-HCFs with addition of TNF-α and IL-4 resulted in increased number of cardiomyocytes in a proliferative state as compared with A-HCF, while co-culture of uA90-HCFs and uA106-HCFs resulted in significantly increased number of cardiomyocytes in a proliferative state.

### <Long-term Therapeutic Effect of Cardiac Fibroblasts with Increased Percentage of CD90- and CD106-positive Cells on Chronic Heart Failure in Rat>

Chronic heart failure model rats prepared by the ischemia-reperfusion described above received intramyocardial administration of A-HCF, uA-HCF, and uA90-HCF shown in Table 1 with a syringe, and then therapeutic effects of various fibroblasts on heart failure in the rats were evaluated by echocardiography. The tests were performed at N = 6 for Sham, N = 4 for Control, N = 7 for A-HCF administered group, N = 8 for uA-HCF administered group, and N = 9 for uA90-HCF administered group. Evaluation of the percentage of CD90- and CD106-positive cells in each type of the prepared fibroblast population with a flow cytometer revealed that there was 1.77% CD90- and CD106-double positive cells (DP) for A-HCF, while 21.36% DP for uA-HCF and 61.25% DP for uA90-HCF. The results are shown in FIGS. 7-1, 7-2, and 7-3.

As can be seen from FIGS. 7-1, 7-2, and 7-3, administration of A-HCF showed no therapeutic effect on heart failure, but uA-HCF and uA90-HCF successfully showed significantly improved LVEF and LVFS in 4 weeks after transplantation.

In addition, a comparative analysis of the percent change of LVEF and LVFS (Delta-LVEF, Delta-LVFS) with various fibroblasts demonstrated that uA-HCF and uA90-HCF showed increased Delta-LVEF and Delta-LVFS as compared with before cell transplantation, and that uA90-HCF showed significantly improved Delta-LVEF and Delta-LVFS as compared with A-HCF.

### <Percentage of G-CSF-positive Cells in Human Fibroblasts and Increase in Percentage of G-CSF-positive Cells with Addition of TNF-α and IL-4>

Cardiac fibroblasts (uA-HCF) with increased percentage of CD90- and CD106-positive cells were prepared as described in Table 2, by addition of a combination of two agents, TNF-α (50 ng/mL) and IL-4 (2 ng/mL) and culture in HFDM-1(+) medium for 3 days. Further, cardiac fibroblasts (uA90·106-HCF) were prepared by cell sorting using an anti-CD90 antibody and an anti-CD 106 antibody. In addition, cardiac fibroblasts (A-HCF), fetal CD106-negative cardiac fibroblasts (F-VNCF), and fetal CD106-positive cardiac fibroblasts (F-VCF) cultured without addition of TNF-α and IL-4 were prepared as controls.

**Table 2. Various fibroblasts used as analysis samples**

| Origin | + TNF-α and IL-4 | Cell Sorting | Abbreviation |
|---|---|---|---|
| Fetal Heart | - | CD106- | F-VNCF |
| | - | CD106+ | F-VCF |
| Adult Heart | - | - | A-HCF |
| | + 50 ng/mL of TNF-α + 2 ng/mL of IL-4 | - | uA-HCF |
| | | CD90+ CD106+ | uA90·106-HCF |

Evaluation of the percentage of G-CSF-positive cells in each type of the fibroblasts by flow cytometry revealed that F-VNCF showed a G-CSF-positive cell percentage of 9.04%, while F-VCF showed 4.35%, A-HCF showed 6.75%, uA-HCF showed 24.70%, and uA90·106-HCF showed 92.50% (FIG. 8). These results revealed that naturally occurring CD106-positive cells (F-VCF) had a low percentage of G-CSF-positive cells, and that CD106-positive and/or CD90-positive cardiac fibroblasts artificially produced by contact with TNF-α and IL-4 showed increased percentage of G-CSF-positive cells as the percentage of CD106- and/or CD90-positive cells increased.

### <Long-term Therapeutic Effect of Cardiac Fibroblasts with Increased Percentage of CD106- and CD90-positive Cells on Chronic Heart Failure in Rat>

Chronic heart failure model rats prepared by ischemia-reperfusion received intramyocardial administration of A-HCF, uA-HCF, and uA90-HCF with a syringe, and then long-term therapeutic effects of the various fibroblasts on heart failure in the rats were estimated by echocardiography. The results showed no therapeutic effect of A-HCF administration on heart failure, and 18 weeks after transplantation the results showed LVEF of 44.1 ± 2.8% and LVFS of 17.8 ± 1.4% (FIGS. 9-1, and 9-2 A and C). uA90-HCF after cell sorting using CD90 antigen as a marker showed effects of improving LVEF and LVFS in long term (after 12 weeks post-transplantation), and 18 weeks after transplantation the results showed LVEF of 61.0 ± 2.2% and LVFS of 27.2 ± 1.4%.

In addition, a comparative analysis of the percent change of LVEF and LVFS (Delta-LVEF, Delta-LVFS) with various fibroblasts demonstrated that the A-HCF administered group showed a Delta-LVEF value of -6.9 ± 2.6% and a Delta-LVFS value of -3.5 ± 1.3% at 18 weeks after transplantation (FIGS. 9-2B and 9-2D). On the other hand, the uA-HCF administered group showed a Delta-LVEF value of -1.8 ± 3.4% and a Delta-LVFS value of -0.6 ± 1.8% at 18 weeks after transplantation. The uA90-HCF administered group showed a Delta-LVEF value of 9.2 ± 2.6% and a Delta-LVFS value of 5.6 ± 1.6% at 18 weeks after transplantation. Thus, it was understood that uA90-HCF allowed for significant improvement of the function of failing myocardial tissues for a long period of time. The details of the primary endpoints (LVEF and LVFS) and the secondary endpoints (LVEDV, LVESV, LVIDd, LVIDs, LVAWd, LVPWTd, and HR) are shown in Tables 3 to 11.

**Table 3. Changes in left ventricular ejection fraction (LVEF) from a rat model of chronic heart failure by administration of various fibroblasts**

| Groups | | LVEF (%) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 51.0 | 47.4 | 45.0 | 43.9 | 45.8 | 47.6 | 45.8 | 46.2 | 45.0 | 44.1 |
| | S.E. | 1.2 | 1.3 | 1.2 | 1.5 | 1.8 | 2.1 | 1.9 | 1.8 | 2.1 | 2.8 |
| uA-HCF | Mean | 51.5 | 50.2 | 52.9 | 55.7 | 56.5 | 56.2 | 53.8 | 53.4 | 53.3 | 49.6 |
| | S.E. | 0.9 | 1.7 | 2.1 | 2.4 | 3.0 | 4.0 | 4.0 | 4.6 | 4.4 | 4.0 |
| uA90-HCF | Mean | 51.8 | 54.2 | 58.3 | 59.6 | 60.0 | 60.9 | 61.2 | 61.5 | 61.3 | 61.0 |
| | S.E. | 0.7 | 1.1 | 1.3 | 0.9 | 1.4 | 1.7 | 1.6 | 1.8 | 2.1 | 2.2 |
| Sham | Mean | 88.7 | 92.6 | 90.8 | 93.4 | 90.9 | 91.1 | 90.6 | 90.3 | 90.2 | 90.5 |
| | S.E. | 0.6 | 0.6 | 0.4 | 1.1 | 0.5 | 0.8 | 0.9 | 0.8 | 0.7 | 0.6 |
| Control | Mean | 52.9 | 48.9 | 47.0 | 44.5 | 42.2 | 39.8 | 38.1 | 39.6 | 39.9 | 38.4 |
| | S.E. | 1.3 | 0.9 | 0.3 | 0.5 | 1.0 | 1.2 | 1.5 | 1.6 | 1.0 | 0.4 |

**Table 4. Changes in left ventricular fractional shortening (LVFS) from a rat model of chronic heart failure by administration of various fibroblasts**

| Groups | | LVFS (%) | | | | | | | | | |
|---|---|---|---|---|---|---|---|---|---|---|---|
| | | Before Adm inistration | 2W | 4W | 6W | 8W | 10W | 12W | 14W | 16W | 18W |
| A-HCF | Mean | 21.2 | 19.3 | 18.1 | 17.6 | 18.5 | 19.5 | 18.5 | 18.7 | 18.1 | 17.8 |
| | S.E. | 0.7 | 0.7 | 0.6 | 0.8 | 0.9 | 1.1 | 1.0 | 0.9 | 1.1 | 1.4 |
| uA-HCF | Mean | 21.5 | 20.8 | 22.3 | 24.0 | 24.5 | 24.6 | 23.2 | 23.1 | 23.0 | 20.9 |
| | S.E. | 0.5 | 0.9 | 1.1 | 1.4 | 1.3 | 2.3 | 2.3 | 2.7 | 2.5 | 2.1 |
| uA90-HCF | Mean | 21.6 | 23.0 | 25.4 | 26.1 | 26.4 | 27.0 | 27.1 | 27.4 | 27.3 | 27.2 |
| | S.E. | 0.4 | 0.6 | 0.8 | 0.5 | 0.8 | 1.0 | 1.0 | 1.1 | 1.3 | 1.4 |
| Sham | Mean | 51.6 | 57.9 | 55.0 | 60.1 | 55.0 | 55.7 | 54.7 | 54.2 | 54.0 | 54.5 |
| | S.E. | 0.9 | 1.2 | 0.8 | 2.2 | 0.9 | 1.5 | 1.4 | 1.3 | 1.1 | 1.0 |
| Control | Mean | 22.2 | 20.1 | 19.1 | 17.8 | 16.7 | 15.6 | 14.8 | 15.5 | 15.7 | 14.9 |
| | S.E. | 0.7 | 0.4 | 0.1 | 0.2 | 0.5 | 0.6 | 0.7 | 0.8 | 0.4 | 0.2 |

**Table 5. Changes in left ventricular end-diastolic volume (LVEDV) from a rat model of chronic heart failure by administration of various fibroblasts**

| Groups | | LVEDV (*µ* L) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 378.9 | 523.0 | 520.0 | 560.6 | 586.1 | 596.6 | 622.7 | 645.3 | 648.6 | 661.9 |
| | S.E. | 29.4 | 26.2 | 23.1 | 32.6 | 32.6 | 22.6 | 34.3 | 33.9 | 35.8 | 37.6 |
| uA-HCF | Mean | 386.9 | 542.5 | 547.8 | 549.9 | 596.3 | 617.4 | 678.9 | 730.5 | 740.5 | 770.3 |
| | S.E. | 23.4 | 41.9 | 50.5 | 57.6 | 62.5 | 51.2 | 69.1 | 94.2 | 88.4 | 86.8 |
| uA90-HCF | Mean | 437.3 | 513.1 | 519.1 | 546.7 | 558.2 | 565.2 | 595.0 | 640.9 | 631.9 | 695.7 |
| | S.E. | 20.0 | 33.1 | 36.7 | 31.4 | 34.5 | 33.6 | 40.1 | 41.4 | 37.7 | 56.8 |
| Sham | Mean | 221.8 | 249.3 | 279.0 | 312.8 | 333.5 | 350.8 | 368.0 | 393.2 | 393.2 | 402.0 |
| | S.E. | 5.9 | 8.2 | 8.8 | 12.4 | 10.7 | 9.2 | 14.4 | 7.4 | 7.4 | 3.4 |
| Control | Mean | 423.3 | 540.3 | 496.3 | 607.3 | 655.3 | 691.3 | 743.3 | 701.5 | 727.5 | 761.3 |
| | S.E. | 46.6 | 22.9 | 41.7 | 33.4 | 60.6 | 81.4 | 103.1 | 65.5 | 50.5 | 44.0 |

**Table 6. Changes in left ventricular end-systolic volume (LVESV) from a rat model of chronic heart failure by administration of various fibroblasts**

| Groups | | LVESV (*µ* L) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 186.0 | 275.9 | 287.3 | 317.1 | 321.0 | 313.4 | 340.7 | 350.7 | 360.4 | 373.7 |
| | S.E. | 16.1 | 18.1 | 17.4 | 25.6 | 27.5 | 19.0 | 29.0 | 29.4 | 31.2 | 35.4 |
| uA-HCF | Mean | 188.6 | 274. 4 | 264.3 | 251.4 | 270.0 | 283.0 | 332.1 | 368.8 | 372.5 | 409.8 |
| | S.E. | 13.9 | 30.5 | 35.7 | 40.2 | 45.3 | 47.5 | 60.2 | 78.3 | 76.6 | 75.8 |
| uA90-HCF | Mean | 210.8 | 234.9 | 217.2 | 221.7 | 224.8 | 223.9 | 233.9 | 252.0 | 249.3 | 279.4 |
| | S.E. | 9.7 | 16.3 | 18.3 | 15.4 | 19.6 | 21.2 | 23.7 | 28.0 | 26.8 | 38.0 |
| Sham | Mean | 25.2 | 18.7 | 25.7 | 21.2 | 30.3 | 31.5 | 35.2 | 38.3 | 38.7 | 38.2 |
| | S.E. | 1.9 | 1.9 | 1.8 | 4.1 | 1.5 | 3.4 | 4.5 | 3.3 | 3.4 | 2.4 |
| Control | Mean | 197.8 | 276.5 | 263.0 | 337.3 | 380.0 | 418.5 | 463.0 | 426.5 | 437.8 | 469.0 |
| | S.E. | 18.3 | 13.7 | 22.6 | 20.7 | 39.2 | 56.2 | 70.2 | 48.8 | 33.3 | 27.3 |

**Table 7. Changes in left ventricular internal diameter at diastole (LVIDd) from a rat model of chronic heart failure by administration of various fibroblasts**

| Groups | | LVIDd (mm) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 7.20 | 8.04 | 8.03 | 8.23 | 8.35 | 8.41 | 8.52 | 8.63 | 8.64 | 8.69 |
| | S.E. | 0.19 | 0.14 | 0.12 | 0.17 | 0.16 | 0.11 | 0.15 | 0.15 | 0.16 | 0.17 |
| uA-HCF | Mean | 7.27 | 8.12 | 8.13 | 8.13 | 8.35 | 8.47 | 8.72 | 8.89 | 8.95 | 9.08 |
| | S.E. | 0.15 | 0.20 | 0.24 | 0.27 | 0.28 | 0.23 | 0.29 | 0.38 | 0.35 | 0.33 |
| uA90-HCF | Mean | 7.57 | 7.98 | 8.00 | 8.15 | 8.21 | 8.24 | 8.38 | 8.59 | 8.55 | 8.81 |
| | S.E. | 0.12 | 0.17 | 0.19 | 0.16 | 0.17 | 0.16 | 0.19 | 0.19 | 0.17 | 0.23 |
| Sham | Mean | 6.05 | 6.29 | 6.53 | 6.78 | 6.93 | 7.05 | 7.16 | 7.33 | 7.33 | 7.38 |
| | S.E. | 0.05 | 0.07 | 0.07 | 0.09 | 0.07 | 0.06 | 0.09 | 0.05 | 0.05 | 0.02 |
| Control | Mean | 7.48 | 8.14 | 7.90 | 8.46 | 8.66 | 8.80 | 9.00 | 8.86 | 8.98 | 9.12 |
| | S.E. | 028 | 0.12 | 022 | 0.15 | 0.27 7 | 0.35 | 0.42 | 028 | 0.21 | 0.18 |

**Table 8. Changes in left ventricular internal diameter at systole (LVIDs) from a rat model of chronic heart failure by administration of various fibroblasts**

| Groups | | LVIDs (mm) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 5.68 | 6.49 | 6.58 | 6.79 | 6.81 | 6.78 | 6.95 | 7.02 | 7.08 | 7.16 |
| | S.E. | 0.17 | 0.15 | 0.14 | 0.19 | 0.20 | 0.14 | 0.20 | 0.20 | 0.21 | 0.23 |
| uA-HCF | Mean | 5.71 | 6.44 | 6.33 | 6.20 | 6.33 | 6.42 | 6.74 | 6.90 | 6.95 | 7.23 |
| | S.E. | 0.14 | 0.23 | 0.28 | 0.31 | 0.35 | 0.36 | 0.42 | 0.52 | 0.49 | 0.45 |
| uA90-HCF | Mean | 5.94 | 6.14 | 5.97 | 6.03 | 6.05 | 6.03 | 6.11 | 6.25 | 6.23 | 6.44 |
| | S.E. | 0.10 | 0.14 | 0.17 | 0.14 | 0.16 | 0.18 | 0.20 | 0.22 | 0.22 | 0.28 |
| Sham | Mean | 2.93 | 2.65 | 2.94 | 2.72 | 3.12 | 3.13 | 3.25 | 3.36 | 3.37 | 3.36 |
| | S.E. | 0.07 | 0.09 | 0.07 | 0.18 | 0.05 | 0.13 | 0.14 | 0.10 | 0.10 | 0.07 |
| Control | Mean | 5.81 | 6.51 | 6.39 | 6.95 | 7.22 | 7.44 | 7.68 | 7.50 | 7.58 | 7.74 |
| | S.E. | 0.18 | 0.11 | 0.18 | 0.14 | 0.26 | 0.34 | 0.40 | 0.29 | 0.19 | 0.16 |

**Table 9. Changes in left ventricular anterior wall thickness at end-diastole (LVAWd) from a rat model of chronic heart failure by administration of various fibroblasts**

| Groups | | LVAWd (mm) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 1.25 | 1.14 | 1.04 | 0.98 | 0.99 | 1.01 | 0.96 | 0.95 | 0.93 | 0.91 |
| | S.E. | 0.02 | 0.07 | 0.06 | 0.05 | 0.05 | 0.04 | 0.03 | 0.03 | 0.05 | 0.04 |
| uA-HCF | M ean | 1.46 | 1.13 | 1.13 | 1.10 | 1.12 | 1.12 | 1.08 | 1.05 | 0.91 | 0.97 |
| | S.E. | 0.08 | 0.03 | 0.06 | 0.07 | 0.10 | 0.11 | 0.11 | 0.08 | 0.08 | 0.09 |
| uA90-HCF | Mean | 1.22 | 1.13 | 1.09 | 1.13 | 1.12 | 1.17 | 1.18 | 1.17 | 1.18 | 1.18 |
| | S.E. | 0.04 | 0.03 | 0.03 | 0.04 | 0.03 | 0.04 | 0.05 | 0.05 | 0.06 | 0.05 |
| Sham | M ean | 1.47 | 1.51 | 1.45 | 1.51 | 1.53 | 1.53 | 1.55 | 1.56 | 1.57 | 1.56 |
| | S.E. | 0.05 | 0.02 | 0.01 | 0.01 | 0.01 | 0.01 | 0.02 | 0.02 | 0.02 | 0.02 |
| Control | Mean | 1.22 | 1.04 | 1.00 | 1.00 | 0.98 | 0.92 | 0.84 | 0.86 | 0.84 | 0.80 |
| | S.E. | 0.06 | 0.03 | 0.02 | 0.02 | 0.04 | 0.05 | 0.02 | 0.02 | 0.02 | 0.03 |

**Table 10. Changes in left ventricular posterior wall thickness at end-diastole (LVPWTd) from a rat model of chronic heart failure by administration of various fibroblasts**

| Groups | | LVPWTd (mm) | | | | | | | | | |
|---|---|---|---|---|---|---|---|---|---|---|---|
| | | Before Administration | 2W | 4W | 6W | 8W | 10W | 12W | 14W | 16W | 18W |
| A-HCF | Mean | 1.42 | 1.44 | 1.36 | 1.39 | 1.43 | 1.38 | 1.44 | 1.37 | 1.41 | 1.30 |
| | S.E. | 0.02 | 0.04 | 0.06 | 0.04 | 0.05 | 0.05 | 0.02 | 0.04 | 0.02 | 0.05 |
| uA-HCF | Mean | 1.49 | 1.50 | 1.44 | 1.52 | 1.49 | 1.45 | 1.51 | 1.35 | 1.38 | 1.37 |
| | S.E. | 0.06 | 0.04 | 0.03 | 0.04 | 0.07 | 0.04 | 0.05 | 0.06 | 0.04 | 0.03 |
| uA90-HCF | Mean | 1.46 | 1.50 | 1.49 | 1.47 | 1.49 | 1.41 | 1.55 | 1.46 | 1.43 | 1.40 |
| | S.E. | 0.03 | 0.03 | 0.03 | 0.03 | 0.05 | 0.06 | 0.06 | 0.05 | 0.02 | 0.04 |
| Sham | Mean | 1.49 | 1.57 | 1.48 | 1.56 | 1.49 | 1.64 | 1.56 | 1.56 | 1.56 | 1.59 |
| | S.E. | 0.04 | 0.03 | 0.02 | 0.03 | 0.02 | 0.09 | 0.02 | 0.05 | 0.05 | 0.06 |
| Control | Mean | 1.38 | 1.44 | 1.42 | 1.40 | 1.50 | 1.36 | 1.20 | 1.38 | 1.28 | 1.42 |
| | S.E. | 0.02 | 0.07 | 004 | 0.02 | 0.02 | 007 | 0.06 | 0.06 | 0.06 | 0.04 |

**Table 11. Changes in heart rate (HR) from a rat model of chronic heart failure by administration of various fibroblasts**

| Groups | | HR (bpm) | | | | | | | | | |
|---|---|---|---|---|---|---|---|---|---|---|---|
| | | Before Administration | 2W | 4W | 6W | 8W | 10 W | 12W | 14W | 16W | 18W |
| A-HCF | Mean | 359.6 | 354.6 | 350.9 | 358.7 | 344.3 | 351.1 | 346.0 | 341.4 | 346.4 | 345.6 |
| | S.E. | 10.3 | 8.0 | 19.0 | 4.6 | 9.9 | 8.7 | 8.0 | 7.8 | 8.0 | 9.3 |
| uA-HCF | Mean | 373.0 | 353.0 | 354.9 | 358.3 | 354.9 | 341.3 | 340.1 | 342.6 | 338.0 | 355.0 |
| | S.E. | 5.7 | 7.0 | 11.3 | 11.7 | 10.9 | 6.6 | 11.2 | 7.1 | 5.2 | 8.5 |
| uA90-H C F | Mean | 369.0 | 360.7 | 342.3 | 354.7 | 354.5 | 338.9 | 348.8 | 344.2 | 355.8 | 347.3 |
| | S.E. | 8.4 | 9.1 | 11.9 | 11.0 | 12.1 | 10.1 | 8.5 | 7.0 | 12.1 | 10.2 |
| Sham | Mean | 356.8 | 372.2 | 357.8 | 363.3 | 365.5 | 357.7 | 354.0 | 355.0 | 358.8 | 355.8 |
| | S.E. | 5.9 | 8.0 | 8.4 | 8.1 | 8.4 | 12.7 | 10.1 | 8.9 | 11.7 | 14.9 |
| Control | Mean | 363.3 | 360.8 | 355.8 | 345.8 | 347.3 | 351.3 | 354.3 | 366.0 | 359.0 | 343.5 |
| | S.E. | 12.3 | 11.3 | 9.5 | 13.0 | 7.4 | 17.5 | 17.5 | 15.0 | 12.5 | 11.8 |

## Claims

1. A method of producing CD106-positive and/or CD90-positive fibroblasts, comprising the step of:
culturing fibroblasts in the presence of at least one selected from the group consisting of TNF-α and IL-4 to increase the number of CD106-positive and/or CD90-positive fibroblasts.

2. The method according to claim 1, wherein the fibroblasts to be cultured is obtained from an adult.

3. The method according to claim 1 or 2, wherein the CD106-positive and/or CD90-positive fibroblasts are positive for CD106 and CD90.

4. The method according to any one of claims 1 to 3, further comprising the step of enriching the CD106-positive and/or CD90-positive fibroblasts.

5. The method according to claim 4, wherein the enrichment is made using an anti-CD 106 antibody and/or an anti-CD90 antibody.

6. A method of producing a G-CSF-positive fibroblast, comprising the step of:
culturing a fibroblast in the presence of at least one selected from the group consisting of TNF-α and IL-4 to increase the expression level of G-CSF and thereby increase the number of G-CSF-positive fibroblasts.

7. The method according to claim 6, wherein the fibroblasts to be cultured is obtained from an adult.

8. The method according to claim 6 or 7, further comprising, after the culturing, the step of enriching the G-CSF fibroblasts.

9. The method according to claim 8, wherein the enrichment is made using an anti-CD 106 antibody and/or an anti-CD90 antibody.

10. A cell population comprising isolated CD106-positive and G-CSF-positive fibroblasts.

11. The cell population according to claim 10, wherein the percentage (number of cells) of G-CSF-positive fibroblasts in fibroblasts is 6.75% or more.

12. A cell population comprising fibroblasts obtained from an adult,
wherein 5% or more of the fibroblasts are positive for CD106.

13. A cell population comprising fibroblasts obtained from an adult,
wherein 7% or more of the fibroblasts are positive for CD90, CD106, and G-CSF.

14. A pharmaceutical composition comprising the cell population according to any one of claims 10 to 13 and a pharmaceutically acceptable excipient.

15. The pharmaceutical composition according to claim 14, wherein the composition is used for improving cardiac functions.
